(19) Europäisches Patentamt
European Patent Office
Office européen des brevets

(11) Publication number: **0 170 345 B1**

# EUROPEAN PATENT SPECIFICATION

(45) Date of publication of patent specification: **30.10.91**

(51) Int. Cl.⁵: **G01N 33/48**, G01N 15/14

(21) Application number: **85302094.9**

(22) Date of filing: **26.03.85**

(54) **Flow cytometry.**

(30) Priority: **28.03.84 US 594077**

(43) Date of publication of application:
**05.02.86 Bulletin 86/06**

(45) Publication of the grant of the patent:
**30.10.91 Bulletin 91/44**

(84) Designated Contracting States:
**BE CH DE FR GB IT LI NL SE**

(56) References cited:
**EP-A- 0 105 614**
**FR-A- 2 113 336**
**US-A- 3 781 112**

**JOURNAL OF HISTOCHEMISTRY AND
CYTOCHEMISTRY, vol. 24, no. 1, 1976;
H.M.SHAPIRO et al.: "Combined blood cell
conting and classification with fluorochrome
stains and flow instrumentation"; pp. 396-411**

**ARCH. PATHOL. LAB. MED., vol. 107, 1983;
pp. 105-116**

(73) Proprietor: **TECHNICON INSTRUMENTS COR-
PORATION(a Delaware corporation)
511 Benedict Avenue
Tarrytown, New York 10591-5097(US)**

Proprietor: **THE MOUNT SINAI SCHOOL OF
MEDICINE
One Gustave L. Levy Place
New York, NY 10029(US)**

(72) Inventor: **Martin, Grace E.
12 Gessner Place
Tarrytown, NY 10591(US)**
Inventor: **Kanter, Robert J.
5 Belair Drive
Old Bethpage, NY 11804(US)**
Inventor: **Kim, Young Ran
100G High Point Drive
Hartsdale, NY 10530(US)**
Inventor: **Ornstein, Leonard
5 Biltom Road
White Plains, NY 10990(US)**
Inventor: **Ansley, Hudson R.
59 Wawayanda Road
Warwick, NY 10990(US)**

(74) Representative: **Wain, Christopher Paul et al
A.A. THORNTON & CO. Northumberland
House 303-306 High Holborn
London WC1V 7LE(GB)**

## Description

This invention relates to flow cytometry, particularly to the use of specific binding assays in flow cytometry systems.

All animal and plant cells have a large variety of protein enzymes which chemically catalyze the various biochemical reactions that are necessary for maintenance, growth and specialized cell functions. Among these enzymes are subsets which are present in only a few, or even only one kind of cell.

Gomori, Proc. Soc. Exp. Biol. Med., 42:23 (1939) and independently Takamatsu, Trans. Soc. Path Japan, 4:277 (1939) first described enzyme-cytochemistry when they realized that if an appropriate substance, or combination of substances, was introduced into a cell where there existed an enzyme that could produce a light-absorbing and insoluble product from that substance or those substances, all cells which contained that enzyme would become selectively colored (or dark) and could easily be distinguished from all other cells which lacked that enzyme by observing such cells with a light microscope. The introduced substances are natural or synthetic substrates for a specific enzyme which, after reaction with that enzyme, either spontaneously becomes light-absorbing and insoluble or become so because of reaction with other introduced reagent substances.

For such methodology to work with live cells, usually the introduced substances must (a) be able to enter the cell; (b) must not be toxic to the cell; (c) must convert to an extremely insoluble form; and (d) the conversion must occur extremely rapidly. Cells are typically a few to tens of micrometers in diameter. Molecules in aqueous solution typically diffuse such distances in fractions of seconds or seconds. Therefore, if, after enzymatic reaction, the conversion to the insoluble form is delayed, it will deposit far from the enzyme and even outside the cell. Since the product concentration diminishes with the cube of the distance it diffuses from site of reaction, its solubility product might not be exceeded at even short distances from the enzymatic site and, therefore, the cell remains unstained unless the product is extremely insoluble.

It has been especially difficult to simultaneously satisfy all of these criteria for live cells, particularly the combination of the permeability and toxicity criteria. For at least this reason, most enzyme-cytochemical methods have been applied to dead cells. In order to be able to distinguish one cell from another, whether stained or unstained, it is important to preserve a modicum of each cell's original external shape and internal structures. For this purpose, various histological and cytological fixing reagents (fixatives) are used which, to various degrees, insolubilize the proteins, nucleic acids and polysaccharide polymers of which cells are constructed. Fixing reagents which are used prior to enzyme-cytochemical staining must not destroy the catalytic chemical activity of the enzyme to be studied (see, Davis, et al, J. Histochem. and Cytochem., 7:291).

Even for fixed cells, where the permeability and toxicity criteria are usually relaxed, it has, nonetheless, been extraordinarily difficult to satisfy, simultaneously, the remaining criteria.

Early enzyme-cytochemical methods, such as those described above, were therefore characterized by staining patterns which were so poorly localized that staining products often precipitated outside as well as inside the cell which originally contained the specific enzyme. A good deal of research effort of cytochemists in the ensuing years has been to develop methods with extremely high rates of conversion of the substrate plus extreme insolubility of the converted (usually colored) product. Methods which cleanly deposit product within a few nanometers of the enzyme are now known. The first truly high resolution dye-based enzyme-cytochemical staining techniques were reported by Holt and O'Sullivan, Proc. Roy. Soc. B., 148:465 (1958);Davis and Ornstein, J. Histochem. and Cytochem., 7:297 (1959); Davis, Proc. Soc. Exp. Biol. Med., 101:90 (1959); Davis, et al, J. Histochem. and Cytochem., 7:291 (1959); and Lehrer and Ornstein, J. Biophys. and Biochem. Cytol., 6:399 (1959).

Specific binding assay techniques have provided extremely useful analytical methods for determining various organic substances of diagnostic, medical, environmental and industrial importance which appear in liquid media at very low concentrations. Specific binding assays are based on the specific interaction between a ligand, i.e. a bindable analyte under determination, and a binding partner therefor. Where one of the ligand and its binding partner is a hapten or antigen and the other is a corresponding antibody, the assay is known as an immunoassay.

Enzymes are among the many labels which are commonly used in the form of a conjugate in which the enzyme is linked to the binding protein with a low molecular weight ligand with a species like that under assay or its binding partner. A substrate is included and reacts with the enzyme, to the extent permitted by the binding partner interaction, to provide a detectable response. In immunoenzyme-cytochemical staining methods, the very high chemical specificity of antibodies to bind selectively to unique molecular sites which are present on or in special subsets of cells is utilized. Such antibodies are either directly, or indirectly attached to enzymes for which high-resolution enzyme-cytochemical methods exist (e.g. peroxidase enzymes, alkaline phosphatase enzymes, etc.). When such an antibody and enzyme are appropriately attached to their target cell, the preparation can then be stained by an appropriate enzyme-cytochemical method, and only the labeled cells will accumulate light-absorbing (colored) product on or in them. Such methods are now widely used. When fixing reagents are used prior to reaction with the cell-specific antibody, it is essential that they do not destroy the antibody-binding sites of the cells to be studied.

One of the classes of enzyme-labeled specific binding techniques used in immunocytology is referred to as the "immunoperoxidase" method, for which there are five basic protocols. Among the following methods, those which bind larger numbers of peroxidase molecules for each molecule of primary antibody bound increase sensitivity by "enzyme amplification". First, in the "direct conjugate" protocol, a peroxidase-antibody conjugate binds directly to a tissue antigen. Second, in the "indirect conjugate" protocol a primary antibody binds the tissue antigen and is, in turn, bound by a peroxidase-secondary antibody (anti-primary antibody) conjugate. Third, the "labeled antigen" protocol is essentially a sandwich technique in which primary antibody binds to both the tissue antigen and to an analogous antigen which has been conjugated with peroxidase. Fourth, in the "enzyme bridge" protocol, primary and secondary antibodies are bound as described above and the secondary antibody is bound by a third antibody which has been conjugated with peroxidase. Fifth, the "peroxidase-anti-peroxidase" protocol is as described for the enzyme bridge protocol with the addition that the peroxidase-tertiary antibody is followed by an anti-peroxidase antibody and excess peroxidase. In each protocol, hydrogen peroxide and a redox chromogen are introduced to exhibit the extent of available or localized peroxidase by enzyme-cytochemical staining and, thus, tissue specific antigen. See, generally, Falini, et al, Arch. Pathol. Lab. Med., 107:105 (1983).

Avidin is a glycoprotein, molecular weight 68,000, with four binding sites that have high affinity for biotin, one of the B vitamin complex. This high affinity binding has been used as an alternative to the labeled antibody methods described above in immunohistology. In the simplest protocol, a biotin-primary-antibody conjugate binds directly to a cellular antigen and is, in turn, bound by an avidin-peroxidase conjugate. In another protocol, the tissue-bound biotinylated primary antibody is bound with unlabeled avidin which, in turn, is bound with peroxidase-labeled biotin. The third such protocol uses an unconjugated primary antibody, a biotinylated secondary antibody and an avidin-biotin-peroxidase complex, from which it derives its connotation as the "ABC method". See, generally, Falini, et al, supra and Guesdon, J-L, et al, J. Histochem. Cytochem., 27:1131-1139 (1979).

One example of the above is described in Hsu, S-M, et al, J. Histochem. Cytochem., 29:577-580 (1981) which relates particularly to the use of the ABC method to identify antigens in formalin-fixed tissues. Assays were manually performed on formalin-fixed, paraffin-embedded thyroids. Serial sections were cut, deparaffinized, and processed through alcohol to water, as in routine tissue processing. The sections were processed by the "ABC" method and the final staining reactions were achieved by incubating the sections with aqueous hydrogen peroxide and diaminobenzidine tetrahydrochloride.

Human white blood cells can be classified as monocytes, polymorphonucleocytes (PMNs) and lymphocytes. There are two principal classes of lymphocytes. The first of these (the thymus-derived cell or T-cell) is immunologically active in effecting cell-mediated responses and the second (the bone marrow derived cell or B cell) is immunologically active in producing antibodies. It is now recognized that T cells are divided into at least several subtypes, termed "helper", "suppressor", and "killer" T cells, which have the function of (respectively) promoting a reaction, suppressing a reaction, or killing (lysing) foreign cells. Lymphocyte subclasses of clinical interest are not easily distinguishable by other than immunological methods. The antigen on the outer surfaces of these lymphocytes are distinguishable with specific antibodies. It is particularly important to recognize and understand that a number of these antigens have been found to be remarkably fragile in the sense that mild chemical or physical treatment of the cell can either destroy the antibody-combining site and/or detach the antigen from the cell surface.

When lymphocytes are stained by immuno-enzyme-cytochemical methods with the cells in liquid suspension, they have typically been stained live, with high resolution methods. These produce a fine stippling of colored product on the outer cell surface. This distribution of product is due to the fact that the insoluble colored product precipitates within a few nanometers of the enzyme molecules (which are all on the outside surface of the cell). Half or more of the product diffuses away from the cell. When this diffused product precipitates, it is mostly freely suspended in the aqueous medium and drifts or is washed away from the cell. The other part diffuses towards the cell and either precipitates between and among the antibody and antigen molecules on the surface of the cell and is trapped in that network, or precipitates on or in the cell membrane, or, if the membrane is permeable to the product, just inside the cell.

Such surface-stippled cells absorb much less light than cells which have a similar amount of stain distributed more uniformly on or within the cells. Binet, et al, Blood Cells, 6:371-376 (1980) have examined such preparations by flow-cytometry (for instrumentation, see U.S. Patent No. 3,740,143). Such surface-stippled cells generated too weak a signal to permit clean separation of the signals of the stained and unstained cells.

Also, it has been found that it is very difficult to get reproducible levels of such staining on duplicate samples of the same live lymphocytes, probably attributable to the variable damage to, and loss of the surface antigens of lymphocytes, and variable changes in permeability which can occur when live cells are manipulated by centrifugation, washing, etc., many times as is often required for such method. Therefore, it would appear that the low and variable staining levels can be in part attributed to the variable damage to, and losses from extensively manipulated live cells.

We have now found that it is possible both to modify the treatment of lymphocytes as well as the composition of the enzyme-cytochemical reagent solution, so that a highly reproducible response is obtained and a substantial quantity of the detectable enzyme product, which is initially produced on the outside surface of the cell, is precipitated on and/or in the cell to produce a large absorption signal which is easily resolved from the signals produced by unstained lymphocytes and other leukocytes in a flow cytometer.

According to the present invention there is provided a flow cytometry method of reproducibly detecting a cell population of interest in a heterogeneous cell suspension, which method comprises:

a) firstly combining said cell suspension with a fixative reagent and thereafter combining the cells so-fixed with a reagent comprising primary binding protein (e.g. primary antibody) specific for said cell population of interest;

b) coupling enzymes to said primary binding protein with at least one ligand;

c) thereafter reacting said fixed cell suspension with a chromogen-containing composition for staining said cell population of interest the chromogen being substantially insoluble in the reaction medium in its reacted form, whereby at least one of the absorption and scattering properties of the cells of said cell population of interest is altered;

d) subsequently passing said reacted cell suspension, substantially a cell at a time, through a light beam while measuring at least said one of said properties of the cells passing therethrough; and

e) differentiating cells of said population of interest based on measurement of at least said one of said properties of said cells of interest.

The antibodies and other reagents used in the method can be combined with a cell sample in a continuous-flow system, as in U.S. Patent No. 3,740,143, supra, or in a discrete reaction chamber at any point upstream of the point of illumination in the flow cytometer.

In accordance with the invention, it has been observed that use of this methodology for the automated determination of different populations, primarily of white blood cells, has the effect of causing larger quantities of the detectable form of the redox chromogen to be retained on the surface and/or in the cells of interest as distinguished from the surrounding medium. Better differentiation of cell populations is, therefore, made possible.

The accompanying drawings (which are referred to hereinafter) illustrate the scattering-absorptive patterns in 2-dimensional plots of individual leukocytes in cell suspensions passed through a flow cytometer, each leukocyte being represented by a black dot.

Figures 1,2 and 4 illustrate results obtained using prior art techniques for processing unfixed leukocyte-enriched cell suspensions. Figure 3 is an unlabeled control for such prior art techniques.

Figures 5 and 6 illustrate the results obtained when fixation of the cell suspension is effected after and before, respectively, the application of the second antibody to the leukocyte. Figure 7 is an unlabeled control for the processes depicted in Figures 5 and 6.

Figures 8 and 10 illustrate the results obtained from a corresponding method according to the present invention when using whole blood, and a corresponding method where cell fixation is effected immediately after the application of the primary antibody to the leukocytes respectively. Figures 9 and 11 are unlabeled controls for Figures 8 and 10, respectively.

The method of the invention is useful in that it permits the detectable species used to selectively and intensely color certain cells, particularly white blood cells, and not others. The cell sample can, for example, be whole blood or a heterogeneous white blood cell sample. The whole blood sample is preferably one in which the red blood cells have been lysed prior to introduction into the flow cytometer.

The terms "specific binding protein" as used herein refers to any substance, or class of substances, which has a specific binding affinity for the cell population of interest to the exclusion of other substances. In the majority of embodiments, the present invention will incorporate specific binding assay reagents which interact with the sample in an immunochemical manner. That is, there will be an antigen-antibody or hapten-antibody relationship between reagents and/or antigens associated with the cells in the cell population of interest. Such assays therefore are termed immunoassays and the special interaction between the ligand and its receptor, or binding partner, is an immunochemical binding. The use of either polyclonal or monoclonal antibodies is contemplated unless otherwise indicated. Additionally, it is well understood in the art that other binding interactions between the differentiating characteristics of the cell surface and a binding partner can serve as the basis of other specific binding assays.

The primary specific binding protein is usually an antibody which is specific for cell surface marker antigens. Such antigens are those which differentiate populations or subpopulations of white blood cells, tumor cells, or other heterogeneous cell samples within which it is useful to distinguish various groups. For example, several antibodies are known to be useful for the differentiation of lymphocyte classes and subclasses, including monoclonal antibodies from various hybridoma cell lines. Such include mouse monoclonal antibodies having designated specificities for all human T cells, suppressor T cells, helper T cells and the like. Other such antibodies are known for specific reactivity with human B cells.

In accordance with the present invention, in order to eliminate, or at least reduce, the damage to and losses from extensively manipulated live lymphocytes, the cells are fixed as early as is feasible, i.e. preceding the application of the antibody. A fixative is chosen which insolubilizes and stiffens the cells without destroying the chemical activity of the cell-specific surface antigen and which does not cause the cells to adhere to one another or to the walls of the vessel in which they are contained. Monoaldehydes, such as formaldehyde, paraformaldehyde and acrolein, and dialdehydes such as glutaraldehyde, used alone or in combination, have been found useful for cells in suspension (see, for example, U.S. Patents 3,741,875 and 4,412,004).

The secondary specific binding protein is usually an antibody against the class of proteins, usually immunoglobulins, of which the primary specific binding protein is a member. Thus, this secondary antibody reacts with and is specific for all antibodies of the primary antibody class.

In one preferred embodiment, the methodology also uses a reagent conjugate comprising an enzyme which is bound to an avidin molecule. Another embodiment of this methodology uses a reagent conjugate comprising an enzyme bound to a biotin molecule which is in turn bound to an avidin molecule. Examples of enzymes suitable for such use include peroxidase (such as horseradish peroxidase), alkaline phosphatase and combinations of these and other enzymes.

The redox chromogen used is critical in that it must be insoluble in the reaction medium in its reacted form. Those which have been identified as useful in the invention include 3-amino-9-ethylcarbazole and 4-chloro-1-naphthol. Chromogens producing a product appreciably soluble in the reaction environment are not useful.

As previously noted in accordance with the invention, the primary antibody, biotinylated secondary antibody, labeled conjugate, enzyme substrate and redox chromogen can be combined with the cell sample under assay, either prior or subsequent to introducing said cell sample to a flow cytometer in which the method is to be performed.

It is preferred to introduce the cell sample into a fluid stream flowing in a conduit or analysis channel in the flow cytometer. This preferably comprises establishing a flowing stream of a flowing fluid sheath stream in the conduit or analysis channel and thereafter introducing the said sample into the flowing fluid sheath stream. Such sheath streams are usually of fluids having a refractive index substantially identical to that of the cell sample suspending medium. One such flow cytometer which uses a sheath stream carrier fluid is used in the Technicon Hemalog D and H-6000 systems, which handle all routine hematology tests. Detailed information on the Hemalog D and H-6000 systems is available from Technicon Instruments Corporation, Tarrytown, NY.

In accordance with the invention, it has been observed that use of this methodology for the automated determination of different populations, primarily of lymphocytes, has the effect of causing the detectable form of the redox chromogen to inhabit and be retained by the lymphocyte population of interest as distinguished from the surrounding medium or the lymphocytes from the sample which are other than those for which the assay method is specific. Better differentiation of cell populations is therefore made possible.

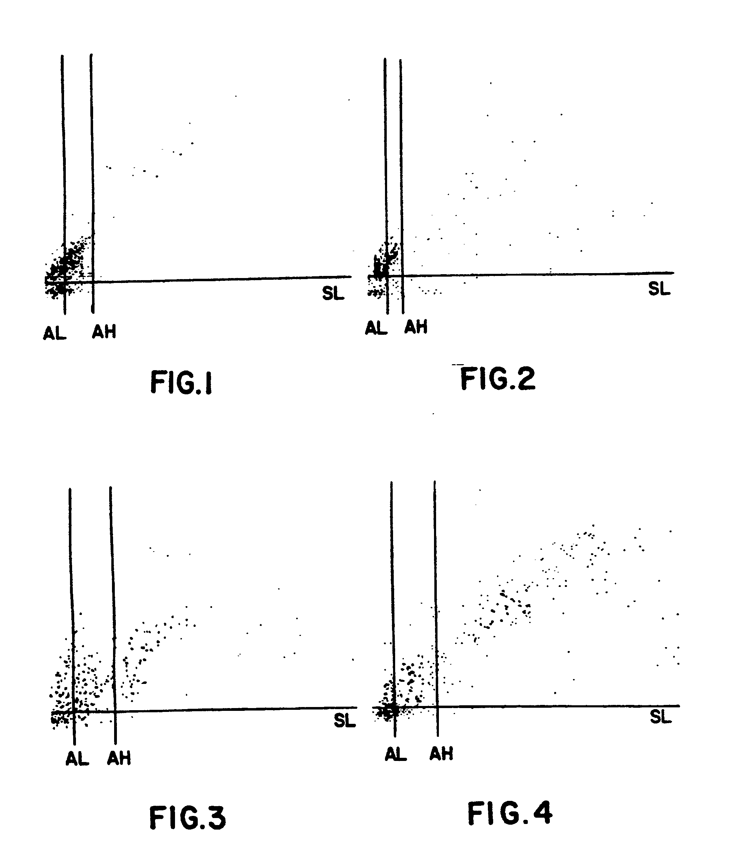

Each of Figs. 1-11 are 2-dimensional displays from the peroxidase channel of an H-6000 instrument system (Technicon Instruments Corporation, Tarrytown, NY) in which absorption is measured along the abscissa and light scattered out of the forward direction is measured along the ordinate. Each dot represents the measured coordinates of a single cell. Three pre-set thresholds enable the operator to separate and count distinct clusters of signals. Absorption Low (AL) and Absorption High (AH) are shown as vertical lines. Scatter Low (SL) is shown as a horizontal line. H-6000 gives separate counts for all signals above SL and to the left of AL, between AL and AH, and to the right of AH. It will ignore all the signals below SL, which represent signals smaller than those from cells, thus removing noise signals due to red cell ghosts, platelets, etc.

These lymphocyte-labeling methods are applied to either lymphocyte-enriched samples or whole blood samples. Whole blood contains both neutrophils and eosinophils (which are PMNs) and monocytes, all of which carry endogenous peroxidases. After peroxidase staining, distinguishing these cells from peroxidase-labeled lymphocytes could pose a problem. As will be illustrated, because of the much greater intensity of staining of eosinophils, neutrophils and some monocytes, the signals from these cells are easily separated from the positive (peroxidase-labeled) lymphocytes by the AH threshold. The remaining weakly stained monocytes which lie between the AL and AH threshholds can be counted separately in the control, and subtracted from the count from between the AL and AH threshholds in the corresponding experimental run.

Also, it is usual for even the best lymphocyte-enriched samples to be contaminated with a few PMNs and monocytes. (See Figs. 1-7.) Similar subtraction of appropriate controls correct for such contamination.

The following Examples are given by way of illustration of the general principle upon which the present invention is based. Example III experiment (b) demonstrates results obtained from an embodiment of the present invention. Standard, commercially available reagent grade chemicals were used whenever possible.

## EXAMPLE I

Several experiments were performed in which the prior art was explored and illustrated with respect to detection and counting of lymphocyte subsets by means of immunoassays using a peroxidase indicator labeling system with flow cytometry in visible and near infrared light. Following the procedure described in the teaching of Binet, et al, supra, an "indirect conjugate" protocol was used in which the mouse monoclonal pan T-cell antibody, T101 (Hybritech, LaJolla, CA), was followed by a peroxidase-conjugated anti-mouse secondary antibody (Cappel, Cochranville, PA). The peroxidase enzyme thus associated with the surface of the T cells was stained by each of one of two different redox chromogens, namely, 3-amino-9-ethyl-carbazole and 4-chloro-1-naphthol. The details of the methods employed are as follows:

Experiment A

Lymphocyte-enriched suspensions were prepared as described in Boyum, A., Scand. J. Clin.

Lab. Invest., 21, Suppl. 97:77 (1968). The lymphocyte fraction was harvested and the cells washed three times by centrifuging for 10 minutes at 400 g in neutral phosphate buffered saline (PBS) containing 0.4% bovine serum albumin (PBS/BSA). The supernatant fluid was aspirated to waste. Sufficient PBS/BSA was added to the final harvest of cells to yield a concentration of $10^7$ cells per ml. A 100 $\mu$l aliquot of this cell suspension was dispensed into a test tube. An equal volume (100 $\mu$l) of T101 at a strength of 10 $\mu$g/ml was added to the tube and incubated for 30 minutes at 4°C. The cells were then washed with PBS/BSA three times by centrifuging for 45 seconds at 100 g, discarding 99% of the supernatant fluid and retaining the cell pellet each time. The cell pellet was then resuspended in residual supernatant and 100 $\mu$l of secondary antibody conjugated with peroxidase (20 $\mu$l/ml) was added and incubated for one hour at 4°C. The wash procedure in PBS/BSA was repeated three times.

The cells were then resuspended and incubated for 10-20 minutes at room temperature in 1 ml of staining mixture consisting of: 2 mg 3-amino-9-ethylcarbazole (AEC) dissolved in 0.5 ml dimethylformamide (DMF) to which was added 9.5 ml 0.05 M acetate buffer (pH 5) plus 50 $\mu$l 3% $H_2O_2$.

A 1 ml volume of the suspension of cells in the staining mixture was then diluted with 2 ml PBS and the cells were introduced into the flow cell of an H-6000 flow cytometer, by-passing the manifold by disconnecting the sample line at the peristaltic pump leading to the flow cell of the peroxidase channel and placing the disconnected sample line directly into, and to the bottom of, the test tube containing the reacted cell suspension. The results are illustrated in Fig. 1.

A negative control was run in which every step was the same as described above except that the primary antibody was omitted. The results for the control are illustrated in Fig. 3. Fig. 3 (control), shows all lymphocytes to the left of AL. The dots between AL and AH are contaminating monocytes. The dots to the right of AH are contaminating neutrophils and eosinophils.

In Fig. 1, the stained lymphocytes are distributed across AL and into the space between AL and AH. All other cells remain is in the control (Fig. 3). The staining is clearly inadequate to separate all of the stained lymphocytes from the unstained lymphocytes. This can be attributed, in part, to the variable damage to and losses from the cell surfaces following the extensive manipulation of these live cells.

Experiment B

Next, exactly the same method as described in Experiment A was followed, except that 2 mg 4-chloro-1-naphthol was substituted in place of AEC in the staining mixture. An attempt was made to conform to the published method although Binet, et al, do not give specific instructions on the details of their staining mixture containing the 4-chloro-1-naphthol. The results are illustrated in Fig. 2 and are comparable to those illustrated in Fig. 1, wherein the stained and unstained lymphocytes cannot be clearly distinguished. A negative control was run in which every step was the same as described above except that the primary antibody was omitted. The results for the control are the same as are illustrated in Fig. 3.

Experiment C

In this experiment, an "enzyme bridge" protocol, using unfixed cells and T101, was followed. This protocol amplifies the amount of peroxidase bound and had not yet been tested in this kind of system at the time of Binet, et al. In this experiment all the steps were the same as in Experiment A except that, instead of peroxidase-conjugated secondary antibody, the remaining steps were the addition of a biotinylated secondary antibody followed by three PBS/BSA washes, followed by ABC complex, and three more PBS/BSA washes. Secondary antibody and ABC provided in a VectaStain Kit (Vector Laboratories, Burlingame, CA), were used in accordance with the manufacturer's instructions. Although the staining is somewhat increased, as shown in Fig. 4, the stained and unstained lymphocytes still cannot be clearly distinguished, as was the case in Figs. 1 and 2.

Experiment D

Binet, et al, sometimes fixed their cells after they had been completely processed (i.e., after staining step with AEC in Example I). Their procedure was to add 1 ml 0.7% formaldehyde to 1 ml of the AEC cell suspension, allowing the mixture to stand at room temperature for 10 minutes and diluting with 1 ml PBS before introduction into an H-6000 flow cell. The results were substantially identical to those illustrated in Fig. 1.

This example shows that the prior art was inadequate for lymphocyte differentiation even when combined with every known improvement in the art. There was no separation of positive and negative lymphocytes into distinct clusters and poor reproducibility was noted for all the attempts described here.

Further, this prior art is characterized by either no fixation or fixation very late in the procedure, long after the cells have been severely abused by the intervening processing steps and yielded inadequately stained cells.

EXAMPLE II

The experiments reported here compare "ABC" lymphocyte differentiation immunoassays using OKT3 (Ortho Diagnostics, Raritan, NJ) or T101 (Hybritech, LaJolla, CA)as the primary antibody, again using a lymphocyte suspension prepared as described in the previous example. In one experiment (Experiment A) a fixation step was performed after incubation with secondary antibody. In a second experiment (Experiment B) the same type of fixation was performed prior to incubation with secondary antibody. In a third experiment (Experiment C), the second experiment is repeated with T101.

Experiment A

A 100 μl aliquot (approximately $10^6$ cells) of a lymphocyte enriched suspension (as in Example I, Experiment A) was dispensed into a clean test tube. A 100 μl volume of phosphate buffered saline containing 0.4% bovine serum albumin (PBS/BSA) and 5 μl OKT3 (pan T primary antibody at a strength of 25 μg/ml, was added and incubated for 15 minutes at 4°C. One (1) ml of PBS/BSA containing 0.3% ethylene diamine tetracetic acid (EDTA) (PBS/BSA/EDTA) washing solution was added to the above and the mixture was centrifuged for 45 seconds in order to pellet the cells without pelleting an excessive number of platelets. 99% of the supernatant was aspirated to waste and this washing procedure (PBS/BSA/EDTA) repeated two more times. This yielded a pellet of washed lymphocytes including OXT3-bound cells.

These cells were then resuspended in residual supernatant and 100 μl of biotin-conjugated anti-mouse immunoglobulin antibody (Vector Labs, Burlingame, CA), diluted according to the Vector protocol, was added. The mixture was incubated for 15 minutes at 4°C. Again, three washes were performed with PBS/BSA/EDTA. This yielded a pellet of washed lymphocytes including OKT3/secondary antibody-biotin bound cells.

Then, after reaction with secondary antibody, the cells were resuspended in residual supernatant and 1.0 ml of 4.6% formaldehyde in 0.07 M PB was added and incubated for 10 minutes at 4°C. The mixture was washed as above three times. This yielded a pellet of washed formaldehyde-fixed lymphocytes including OKT3/secondary antibody-biotin bound cells.

These cells were then resuspended in residual supernatant and incubated with 100 μl (90-190 μg ml) avidin-biotin-horseradish peroxidase complex

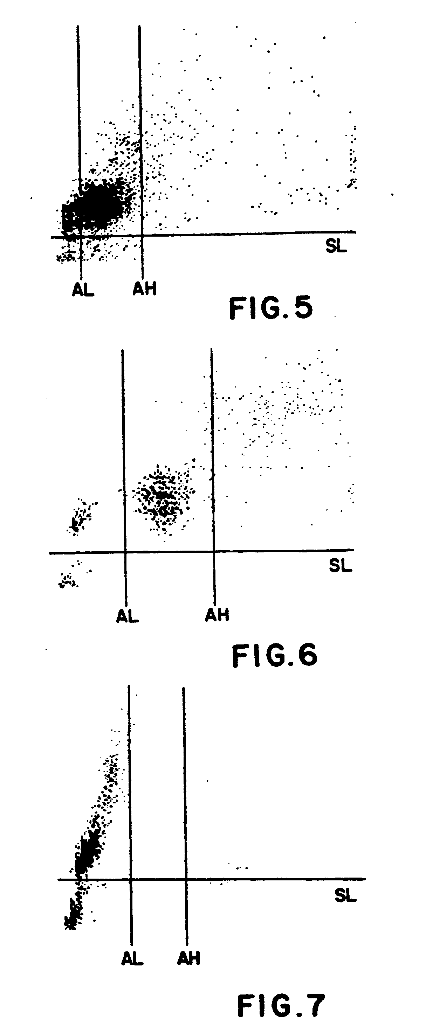

(ABC) at 4°C for 15 minutes. Again, three washes were performed using PBS/BSA/EDTA. This yielded a pellet containing fixed peroxidase-bearing cells. This pellet was resuspended in residual supernatant and a 1.0 ml aliquot of a staining solution was added. The staining solution contained 8 mg 3-amino-9-ethylcarbazole (AEC) in 10 ml methanol combined with 100 $\mu$l 3% $H_2O_2$ made up to 25 ml using 0.05 M sodium acetate buffer (pH 5). After 10 minutes at room temperature, the mixture was combined with 2.0 ml PBS and aspirated directly through a sheathed H-6000 flow cell via a peristaltic pump to obtain the cell signatures in the peroxidase channel. The result is illustrated in Fig. 5. A negative control was run in which every step was the same as described above except that the primary antibody was omitted. The results for the control are illustrated in Fig. 7.

Note that some separation between unstained lymphocytes (to the left of AL) and the stained lymphocytes (between AL and AH) has been achieved in this protocol which fixes the cells after reaction with this second antibody. Such separation is marginal.

Experiment B

A 100 $\mu$l aliquot of a lymphocyte-enriched suspension was treated as described in Experiment A to yield a pellet of washed lymphocytes including OKT3-bound cells.

These cells were then resuspended in residual supernatant and 1.0 ml of 4.6% formaldehyde in 0.07 M PB was added and incubated for 10 minutes at 4°C. The mixture was washed with PBS/BSA/EDTA three times. This yielded a pellet of washed, formaldehyde-fixed lymphocytes including fixed primary antibody (OKT3)-bound cells.

Then, these cells were reacted with biotin-conjugated anti-mouse immunoglobulin antibody, ABC complex, and the staining solution as described in Experiment A. After 10 minutes at room temperature, the mixture was combined with 2.0 ml PBS and fed into an H-6000 flow cytometer as described above. The result is illustrated in Fig. 6.

Experiment C

An experiment identical to experiment B was run, except that T101 was used instead of OKT3, as the primary antibody. The results were substantially the same as those obtained in Experiment B, Fig. 6.

Thus, Experiments A, B and C demonstrate that the labeled lymphocyte subset no longer forms a continuum with the unlabeled subset, as in Example 1, Figs. 1, 2 and 4. Instead, the labeled subset forms a discrete cluster, separated from the unlabeled cluster by a gap. This gap is narrow in Fig. 5 (post-secondary-antibody fixation), but in Fig. 6 it is very wide (pre-secondary-antibody fixation), thus meeting the essential condition necessary for convenient and accurate counting and classifying of subsets of cells in flow cytometry. The width of the gap can be attributed to how early in the process the cells were fixed.

EXAMPLE III

As reported here, lymphocyte differentiation immuno-assays were also performed in accordance with the invention on whole blood samples using OKT4 (Ortho Diagnostics, Raritan, NJ) or Coulter Clone T4 (Coulter, Hialea, FL) as the primary antibody. In one experiment (Experiment A) a fixation step was performed after incubation with primary antibody (OKT4) but prior to incubation with secondary antibody. In a second experiment (Experiment B) using the method according to the present invention, the same type of fixation was performed prior to incubation with primary antibody (Coulter Clone T4).

Experiment A

A 100 $\mu$l aliquot of anticoagulated whole blood was dispensed into a clean test tube. This was mixed with 100 $\mu$l of cold isotonic buffered saline containing approximately 2.5 $\mu$g/ml OKT4 T-cell monoclonal antibody (Ortho Diagnostics, Raritan, NJ) and the mixture was incubated at 4°C for 15 to 30 minutes. Red cells were then lysed by adding 2 ml of 0.85% $NH_4Cl$ solution at room temperature with thorough mixing. The cell suspension was centrifuged for 1 minute (1,000 g) to harvest the white cells. The white cells were recovered and washed twice in PBS/BSA/EDTA at 4°C. This yielded a pellet of washed white blood cells.

Then, these cells were resuspended in the residual supernatant and fixed by adding 1 ml of 0.075 M phosphate buffered 7.5% formaldehyde solution (pH 6.7) containing 15% dextrose. After 5-10 minutes, the fixed cells were washed twice with PBS/BSA/EDTA. Then, after fixation, the cells were resuspended in residual supernatant, incubated with 0.1 ml of biotinylated secondary anti-mouse immunoglobulin antibody (12.5 $\mu$g/ml) for 15-30 minutes at room temperature and were washed twice with PBS/BSA/EDTA. The washed cells were resuspended in residual supernatant and incubated with 0.1 ml avidin-horseradish peroxidase conjugate (A:HRP) in PBS (50-100 $\mu$g A:HRP per ml PBS). After 15-30 minutes at room temperature, the cells were washed three times with PBS/BSA/EDTA and mixed with 0.5 ml of a staining solution which contained 0.3 mg 4-chloro-1-naphthol, 16%

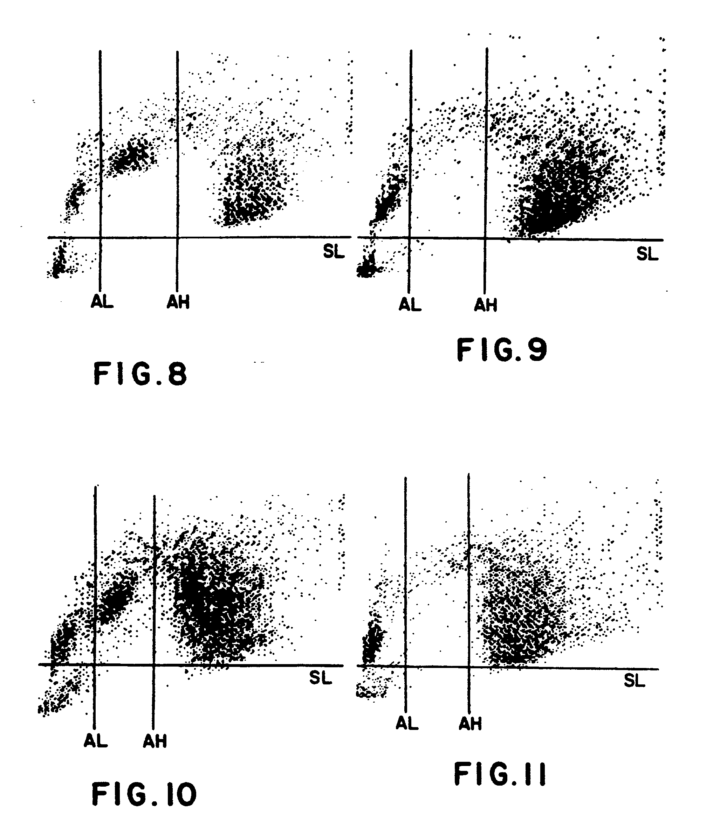

ethanol, 0.01% $H_2O_2$ in 0.025 M phosphate buffer (pH 7.5) and incubated for 10 minutes at room temperature. The stained cell suspension was diluted to 1 ml with phosphate buffer and aspirated directly through the H-6000 flow cell as previously described, with the results illustrated in Fig. 8. A negative control was run in which every step was the same, as described above, except that the primary antibody was omitted. The results for the control are illustrated in Fig. 9.

Experiment B

A 100 $\mu$l aliquot of anti-coagulated whole blood was treated with 0.85% $NH_4Cl$ to lyse red cells, the white cells were harvested and washed twice with PBS/BSA/EDTA as described in Experiment A herein.

Then, the cells were fixed at room temperature by mixing the resuspended pellet with 1 ml of 0.075 M phosphate buffered 7.5% formaldehyde solution (pH 6.7), which contains 15% dextrose, for 5-10 minutes, and washed twice with PBS/BSA/EDTA. The cell pellet was resuspended in residual supernatant, incubated with 0.2 ml of T4-biotinylated primary antibody solution (Coulter, Hialea, FL) in PBS, diluted from stock according to the manufacturer's directions, for 15-30 minutes at room temperature and washed twice with PBS/BSA/EDTA. The resulting pellet was resuspended and incubated with 0.1 ml avidin-peroxidase (50-100 $\mu$g/ml PBS) for 15-30 minutes at room temperature and washed twice with PBS/BSA/EDTA. The resulting pellet was resuspended, mixed with 0.5 ml of a staining solution which contained 0.3 mg 4-chloro-1-naphthol, 0.01% $H_2O_2$ and 16% ethanol (or methanol) in 0.025 M phosphate buffer (pH 7.5) and incubated for 10 minutes at room temperature. The mixture was then combined with 2.0 ml PBS and aspirated directly through a sheathed H-6000 flow cell, as previously described, with the results illustrated in Fig. 10. A negative control was run in which every step was the same as described above except that the primary antibody was omitted. The results for the control are illustrated in Fig. 11.

These experiments demonstrate that the same excellent separation and reproducibility can be obtained in whole blood as in lymphocyte-enriched suspension. That is to say, two lymphocyte subsets, one of which is labeled, form discrete and separate clusters. The PMNs are to the right of AH threshhold. Figs. 9 and 11 show that significant numbers of monocytes fall between AL and AH. These are subtracted from the counts from between AL and AH in Figs. 8 and 10, respectively, to determine the number of stained lymphocytes.

In these whole blood methods, as in Example II, Experiment B (Fig. 6), the early fixation again provides the large and useful gap between unstained and stained lymphocytes.

## Claims

1. A flow cytometry method of reproducibly detecting a cell population of interest in a heterogeneous cell suspension, which method comprises:

   a) firstly combining said cell suspension with a fixative reagent and thereafter combining the cells so-fixed with a reagent comprising primary binding protein specific for said cell population of interest;

   b) coupling enzymes to said primary binding protein with at least one ligand;

   c) thereafter reacting said fixed cell suspension with a chromogen-containing composition for staining said cell population of interest the chromogen being substantially insoluble in the reaction medium in its reacted form, whereby at least one of the absorption and scattering properties of the cells of said cell population of interest is altered;

   d) subsequently passing said reacted cell suspension, substantially a cell at a time, through a light beam while measuring at least said one of said properties of the cells passing therethrough; and

   e) differentiating cells of said population of interest based on measurement of at least said one of said properties of said cells of interest.

2. A method according to claim 1, wherein the coupling of enzymes to said primary antibody is effected with a low-molecular weight ligand and before combining said primary antibodies with said cell suspension.

3. A method according to claim 1 or 2, wherein said cell suspension comprises a heterogeneous white blood cell suspension substantially free of red blood cells, or the cell suspension comprises whole blood in which the red blood cells have been lysed at least prior to step (d) thereof.

4. A method according to claim 1 or 2, wherein in step (a) the primary binding protein is a polyclonal or monoclonal antibody, and/or the primary binding protein is an antibody-biotin conjugate.

5. A method according to any of claims 1 to 4, wherein said cell suspension is fixed with an aldehyde, preferably with formaldehyde.

6. A method according to claim 4, wherein step (b) comprises adding avidin-biotin-enzyme conjugate to said cell suspension, such as an avidin-biotin-peroxidase conjugate or an avidin-biotin-alkaline phosphatase conjugate.

7. A method according to claim 4, wherein the primary binding protein is an antibody-biotin conjugate and step (b) comprises combining the cell suspension with an enzyme-avidin conjugate such as peroxidase-avidin conjugate or an alkaline phosphatase-avidin conjugate.

8. A method according to any of claims 1 to 3, wherein step (b) comprises combining the fixed, antibody bound cells after step (a) with a biotinylated secondary antibody, and optionally then an enzyme-labeled conjugate.

9. A method according to any of claims 1 to 8, wherein said one composition of step (c) contains the chromogen 3-amino-9-ethylcarbazole or 4-chloro-1-naphthol.

**Revendications**

1. Un procédé de cytométrie à écoulement pour détecter de façon reproductible une population de cellules intéressante dans une suspension hétérogène de cellules , procédé comprenant
    a) premièrement la combinaison de ladite suspension de cellules avec un réactif de fixation et ensuite la combinaison des cellules ainsi fixées avec un réactif qui comprend la protéine de fixation primaire spécifique de ladite population de cellules intéressantes;
    b) le couplage des enzymes de ladite protéine de fixation primaire avec au moins un ligand;
    c) ensuite la réaction de ladite suspension de cellules fixées avec une composition contenant un chromogène afin de colorer la population de cellules intéressantes , le chomogène étant complètement insoluble dans le milieu réactionnel après réaction, de telle sorte qu'au moins une des propriétés d'absorption ou de diffusion des cellules de ladite population de cellules intéressantes est altérée;
    d) passage subséquent de la suspension de cellules ayant subi la réaction, sensiblement une seule cellule à la fois, à travers un faisceau de lumière tandis que l'on mesure ainsi une desdites propriérés des cellules qui passent à travers; et

    e) différenciation des cellules de ladite population intéressante basée sur la mesure d'au moins une desdites propriétés desdites cellules intéréssantes.

2. Un procédé selon la revendication 1, où le couplage des enzymes audit anticorps primaire est effectué à l'aide d'un ligand de bas poids moléculaire et avant la combinaison dudit anticorps primaire avec ladite suspension de cellules.

3. Un procédé selon la revendication 1 ou 2, où ladite suspension de cellules comprend une suspension hétérogène de globules blancs suspension complétement débarassée de globules rouges, ou bien la suspension de cellules comprend le sang entier dans lequel les globules rouges ont étés lysés au moins avant l'étape (d)

4. Un procédé selon l'une quelconque des revendications 1 ou 2 où dans l'étape (a) la protéine de fixation primaire est un anticorps polyclonal ou monoclonal, et/ou la protéine de fixation primaire est un conjugué de l'anticorps de la biotine.

5. Un procédé selon l'une quelconque des revendications 1 à 4, où ladite suspension de cellules est fixée à l'aide d'un aldéhyde, de préférence le formaldéhyde.

6. Un procédé selon la revendication 4 où l'étape (b) comprend l'addition d'un conjugué de l'enzyme avidine-biotine à ladite suspension de cellules , tel que le conjugué de l'avidine-biotine peroxydase ou le conjugué de l'avidine-biotine phosphatase alcaline.

7. Un procédé selon la revendication 4 où la protéine de fixation primaire est un conjugué de l'anticorps de la biotine et l'étape (b) comprend la combinaison de la suspension de cellules avec un conjugué de l'avidine-enzyme tel que le conjugué de l'avidine-peroxydase ou le conjugué de l'avidine-phosphatase alcaline.

8. Un procédé selon l'une quelconque des revendications 1 à 3, où l'étape (b) comprend la combinaison des celules fixées attachées à l'anticorps après l'étape (a) avec un anticorps secondaire modifié par la biotine , et en option avec un conjugué d'enzyme marqué.

9. Un procédé selon l'une quelconque des revendications 1 à 8,où ladite composition de l'étape (c) contient le chromogène 3-amino-9-éthylcar-

·bazole ou 4-chloro-1-naphtol.

**Patentansprüche**

1. Durchflußcytometry-Verfahren mit reproduzierbarem Erfassen einer interessierenden Zellpopulation in einer heterogenen Zellsuspension, wobei man
    a) zunächst die Zellsuspension mit einem Fixierungsreagenz und danach die auf diese Weise fixierten Zellen mit einem Reagenz vereinigt, das ein primäres Bindungsprotein enthält, das für die interessierende Zellpopulation spezifisch ist;
    b) Enzyme an das primäre Bindungsprotein mit mindestens einem Liganden kuppelt;
    c) danach die Suspension der fixierten Zellen mit einer ein Chromogen enthaltenden Zusammensetzung zum Anfärben der interessierenden Zellpopulation umsetzt, wobei das Chromogen in seiner umgesetzten Form praktisch in dem Reaktionsmedium unlöslich ist, wodurch die Absorptions- und bzw. oder Streueigenschaften der Zellen der interessierenden Zellpopulation verändert werden;
    d) anschließend die umgesetzte Zellsuspension praktisch Zelle für Zelle durch einen Lichtstrahl hindurchleitet, während mindestens eine der genannten Eigenschaften der hindurchgeleiteten Zellen gemessen wird; und
    e) die Zellen der interessierenden Population aufgrund der Messung mindestens einer der genannten Eigenschaften der interessierenden Zellen voneinander unterscheidet.

2. Verfahren gemäß Anspruch 1, wobei das Kuppeln von Enzymen an den primären Antikörper mit einem Liganden von niedrigem Molekulargewicht und vor dem Vereinigen der primären Antikörper mit der Zellsuspension durchgeführt wird.

3. Verfahren gemäß Anspruch 1 oder 2, wobei die Zellsuspension eine heterogene Suspension von weißen Blutkörperchen, die praktisch frei von roten Blutkörperchen ist, umfaßt, oder die Zellsuspension Gesamtblut umfaßt, in dem die roten Blutkörperchen mindestens vor Stufe (d) lysiert worden sind.

4. Verfahren gemäß Anspruch 1 oder 2, wobei in Stufe (a) das primäre Bindungsprotein ein polyclonaler oder monoclonaler Antikörper und bzw. oder das primäre Bindungsprotein ein Antikörper/Biotin-Konjugat ist.

5. Verfahren gemäß einem der Ansprüche 1 bis 4, wobei die Zellsuspension mit einem Aldehyd, vorzugsweise mit Formaldehyd, fixiert wird.

6. Verfahren gemäß Anspruch 4, wobei Stufe (b) die Zugabe von Avidin/Biotin/Enzym-Konjugat zu der Zellsuspension, wie beispielsweise von einem Avidin/Biotin/Peroxydase-Konjugat oder einem Avidin/Biotin/alkalische-Phosphatase-Konjugat, umfaßt.

7. Verfahren gemäß Anspruch 4, wobei das primäre Bindungsprotein ein Antikörper/Biotin-Konjugat ist und Stufe (b) das Vereinigen der Zellsuspension mit einem Enzym/Avidin-Konjugat, wie einem Peroxydase/Avidin-Konjugat oder einem alkalische-Phosphatase/Avidin-Konjugat, umfaßt.

8. Verfahren gemäß einem der Ansprüche 1 bis 3, wobei Stufe (b) das Vereinigen der fixierten, an Antikörper gebundenen Zellen nach Stufe (a) mit einem biotinylierten sekundären Antikörper und gewünschtenfalls anschließend mit einem mit einem Enzym markierten Konjugat umfaßt.

9. Verfahren gemäß einem der Ansprüche 1 bis 8, wobei die Zusammensetzung von Stufe (c) als Chromogen 3-Amino-9-Ethylcarbazol oder 4-Chlor-1-Naphthol enthält.

FIG.1

FIG.2

FIG.3

FIG.4

FIG.5

FIG.6

FIG.7

SL

AL          AH

## FIG.8

SL

AL          AH

## FIG.9

SL

AL          AH

## FIG.10

SL

AL          AH

## FIG.11